# EUROPÄISCHE PATENTSCHRIFT

(11) **EP 1 242 140 B1**
(45) Veröffentlichungstag und Bekanntmachung des Hinweises auf die Patenterteilung: **08.03.2006**
(21) Anmeldenummer: 00993380.5
(22) Anmeldetag: 05.12.2000
(51) Int. Cl.: A61M 37/00, A61M 5/00, A61M 36/12

(54) **Implantatspritze**
Implant syringe
Seringue pour implant

(30) Priorität: 18.12.1999 DE 19961197
(43) Veröffentlichungstag der Anmeldung: 25.09.2002
(73) Patentinhaber: GAPLAST GmbH, D-82442 Altenau (DE)
(72) Erfinder: KNEER, Roland, 82499 Farchant (DE)
(74) Vertreter: Flosdorff, Jürgen
(86) Internationale Anmeldenummer: PCT/DE2000/004329
(87) Internationale Veröffentlichungsnummer: WO 2001/043811

(56) Entgegenhaltungen:
- FR-A- 2 756 493
- US-A- 4 597 753
- US-A- 5 810 769
- US-A- 5 853 390

## Beschreibung

Die Erfindung betrifft eine Implantatspritze mit einer Spritzennadelanordnung, die eine Spritzennadel, ein Griffstück und ein Präparataufnahmeteil enthält, die zur gemeinsamen axialen Bewegung in einer Führungseinrichtung sitzen, und mit einem an einer Endplatte befestigten Kolben, dessen Vorschub mit Hilfe einer an der Endplatte befestigten und den rückwärtigen Endabschnitt des Kolbens umgebenden Hülse und eines Anschlags an der Führungseinrichtung derart begrenzt ist, daß zwischen der Austrittsöffnung der Spritzennadel und dem Kopfende des Kolbens ein Abstand verbleibt, der mindestens gleich der Länge eines abzugebenden Präparats ist, wobei die Spritzennadel nach Aufhebung einer Blockiereinrichtung mittels des Griffstücks mindestens um eine mit der Länge des Präparats übereinstimmende Strecke zurückziehbar ist.

Eine derartige Implantatspritze wird dazu verwendet, ein vorzugsweise langgestrecktes, strangförmiges Präparat mit einem Langzeitwirkstoff in den Körper eines Patienten abzugeben. Meist wird das Langzeitpräparat in die Bauchdecke eines Patienten gelegt, in die zuvor mittels der Spritzennadel ein Aufnahmekanal für das Präparat eingestochen wurde.

Aus der DE 197 34 385 C1 ist eine Implantatspritze bekannt. Diese Implantatspritze wird so gehandhabt, daß zunächst der Einstichkanal ausgebildet wird, indem die Spritzennadel soweit beispielsweise in die Bauchdecke eines Patienten eingestochen wird, bis die vordere Stirnkante einer zweiteiligen Führungshülse der Implantatspritze auf der Haut des Patienten aufliegt. Nach Abnahme eines Distanzelementes wird anschließend der Kolben vorgeschoben, indem eine Basisplatte eines Abstandhalters der Implantatspritze hintergriffen und Druck auf eine mit dem Kolben verbundene Endplatte ausgeübt wird. Hierbei schiebt der Kolben das Präparat in die Spritzennadel vor, bis eine mit dem Kolben verbundene Hülse auf die Basisplatte des Abstandhalters auftrifft.

Anschließend wird die Implantatspritze an dem vorderen Griffstück und der Basisplatte des Abstandhalters ergriffen, und das Griffstück wird bis zu einem Anschlag zurückgezogen. Beim Auftreffen auf den Anschlag ist die Spritzennadel vollständig von dem Präparat zurückgezogen, das hiermit in dem Einstichkanal abgelegt ist, woraufhin die Implantatspritze entfernt wird.

Mit dieser Implantatspritze läßt sich ein Präparat - das nicht unbedingt eine langgestreckte Strangform haben muß - exakt in den Einstichkanal ablegen, wobei dieser Vorgang - abgesehen von der Ausbildung des Einstichkanals - vollkommen schmerzfrei erfolgt. Ein Nachteil der bekannten Implantatspritze besteht darin, daß zum Zurückziehen der Spritzennadel ein Umgreifen erforderlich ist, wodurch die Handhabung der Spritze erschwert ist.

Aus der FR-A-2 756 493 ist eine Implantatspritze gemäß dem Oberbegriff des Patentanspruchs 1 bekannt. Bei der vorbekannten Implantatspritze ist die Blockiereinrichtung für die Spritzennadelanordnung durch eine Hülse gebildet, die zwischen einer Endplatte, an der der Kolben befestigt ist, und der zugewandten Stirnfläche des Griffstücks angeordnet ist. Um die Spritzennadel z.B. aus der Bauchdecke des Patienten unter Zurücklassung des Präparats zurückziehen zu können, muß diese Hülse entfernt werden. Hierdurch ist die Handhabung der Spritze erschwert und die Dauer der Verabreichung des Präparats verlängert.

Der vorliegenden Erfindung liegt die Aufgabe zugrunde, eine Implantatspritze der eingangs genannten Art so weiter zu entwickeln, daß sie leichter handhabbar ist.

Diese Aufgabe wird erfindungsgemäß durch die Merkmale des Patentanspruchs 1 gelöst.

Vorteilhafte Ausgestaltungen der Erfindung sind in den Unteransprüchen gekennzeichnet.

Bei der erfindungsgemäßen Implantatspritze ist die Spritzennadelanordnung aus Spritzennadel, Griffstück und Präparataufnahmeteil solange von einer Blockierungseinrichtung fixiert, bis der Kolben bei seinem Vorschub den Anschlag erreicht. Die Blockierungseinrichtung hat wenigstens einen, bevorzugt zwei diametral gegenüberliegende Federarme, der bzw. die im unbelasteten Ausgangszustand die Rückwärtsbewegung der Spritzennadelanordnung verhindert bzw. verhindern. Hierzu ragen die bevorzugt zwei Federarme ins Innere der Führungseinrichtung, in der die Spritzennadelanordnung sitzt, wobei sie bevorzugt an einer rückwärtigen Stirnwand der Spritzennadelanordnung anliegen. Der Kolben ist an einer Endplatte befestigt ist, die mit einer den rückwärtigen Endabschnitt des Kolbens umgebenden Hülse versehen ist, die bevorzugt einstückig an der Endplatte angeformt ist. Beim Vorschub des Kolbens läuft die Hülse bevorzugt kurz vor dem Erreichen des Anschlags auf die schräg nach innen ragenden Federarme auf und drückt diese radial nach außen, wodurch die Spritzennadelanordnung zum Zurückziehen freigegeben ist.

Die erfindungsgemäße Ausbildung hat zur Folge, daß die Implantatspritze mit einer gleichbleibenden Griffstellung so betätigt werden kann, daß zunächst der Kolben vorgeschoben und direkt anschließend die Spritzennadelanordnung bis zu einem Anschlag zurückgezogen wird. Hierzu wird das seitlich abstehende Griffstück der Implantatspritze (nach Ausbildung des Einstichkanals) beispielsweise mit Zeigefinger und Mittelfinger hintergriffen, während auf eine mit dem Kolben verbundene Endplatte mittels des Daumens Druck ausgeübt wird, durch den der Kolben bis zu dem hierfür vorgesehenen Anschlag vorrückt. Kurz vor Erreichen des Anschlags oder gleichzeitig mit dessen Erreichen wird die Blokierung der Spritzennadelanordnung aufgehoben, so daß -bei gleicher Griffhaltung- das Griffstück nun auf die Endplatte zu bewegt wird, wobei die Spritzennadel aus dem Einstichkanal unter Zurücklassung des Präparats zurückgezogen wird. Hierbei sollte die Implantatspritze mit der vorderen Stirnkante ihres Gehäuses in Kontakt mit dem Körper des Patienten bleiben.

Dadurch, daß bei der Handhabung der Implantatspritze zwischen dem Vorschub des Kolbens und dem Zurückziehen der Nadel nicht umgegriffen werden muß, ist die Handhabung der Implantatspritze erheblich vereinfacht. Außerdem ist die Implantatspritze -wegen der Beibehaltung einer einzigen Griffstellung- bei der Handhabung stabiler.

Als Führungseinrichtung ist bevorzugt eine Führungshülse vorgesehen, in der die Anordnung aus Spritzennadel, Griffstück und Präparataufnahmeteil sitzt. Die Spritzennadel sitzt bevorzugt fest in einer rohrförmigen Nadelhalterung, die einstückig mit dem Griffstück ausgebildet sein kann.

Der wenigstens eine Federarm ist zweckmäßigerweise einstückig mit der Führungshülse ausgebildet. Der wenigstens eine Federarm kann eine aus der Hülsenwand freigeschnittene schmale Lasche sein, die (im unbelasteten Zustand) in einem spitzen Winkel ins Innere der Führungshülse ragt, wobei ihr freies Ende der Spritzennadelanordnung zugewandt ist.

Weitere Einzelheiten der Erfindung ergeben sich aus der nachfolgenden Beschreibung einer bevorzugten Ausführungsform der erfindungsgemäßen Implantatspritze sowie anhand der Zeichnung. Dabei zeigen:
- Fig. 1: einen Längsschnitt durch die Implantatspritze;
- Fig. 2: eine Einzelheit B der Implantatspritze in einem vergrößerten Maßstab;
- Fig. 3: eine Einzelheit C der Implantatspritze in einem vergrößerten Maßstab und
- Fig. 4: eine Seitenansicht der Implantatspritze.

Die Implantatspritze 1 enthält eine langgestreckte, querschnittlich kreisrunde Führungshülse 2, in der eine Spritzennadelanordnung sitzt, die aus folgenden Bestandteilen zusammengestzt ist: eine Spritzennadel 3, die fest in einer rohrförmigen Nadelhalterung 4 sitzt, die einstückig an einem Griffstück 5 angeformt ist, das die Führungshülse 2 umgibt (Fig. 4) und mit zwei Stegen längsgerichtete Schlitze der Führungshülse 2 durchgreift, wobei diese Stege die Verbindung zu dem Nadelhalter 4 bilden. Ein Präparataufnahmeteil 6 sitzt mit seinem vorderen Endabschnitt in einem Aufnahmeraum 7 hinter dem Nadelhalter 4, wobei der mittige Aufnahmekanal 8 für das nicht dargestellte Präparat mit der Spritzennadel 3 fluchtet. Den axial rückwärtigen Abschluß der Spritzennadelanordnung bildet eine Kappe 9, die eine hintere Stirnwand 10 aufweist. Die Bauteile 3, 4, 5, 6 und 9 sind zur gemeinsamen axialen Bewegung miteinander verbunden.

Die Implantatspritze 1 enthält ferner einen rückwärtigen Kolben 11, der in einer hinteren Endwand 12 mittig verankert ist und eine zentrale Bohrung in der rückwärtigen Stirnwand 10 durchgreift. Der Kolben 11 fluchtet mit dem Kanal 8 des Präparataufnahmeteils 6 und mit der Spritzennadel 3.

Die Endwand 12 ist einstückig mit einer den hinteren Endabschnitt des Kolbens 11 umgebenden Hülse 13 verbunden. Zwischen der vorderen Randkante 14 der Hülse 13 und einem nach außen weisenden Bund 15 der Führungshülse 2 ist vor der Benutzung der Implantatspritze 1 ein Distanzstück 16 angeordnet, das einen sich über seine gesamte Länge erstreckenden Schlitze 17 hat und verhindert, daß der Kolben 11 vorgeschoben wird.

Aus der Wand der Führungshülse 2 sind zwei Federarme 18 freigeschnitten, die in einem spitzen Winkel nach innen ragen und mit ihren Kopfenden 19 an der Stirnwand 10 der Spritzennadelanordnung anliegen. In der in den Figuren dargestellten Position blockieren die Federarme die Spritzennadelanordnung, so daß diese nicht zurückziehbar ist.

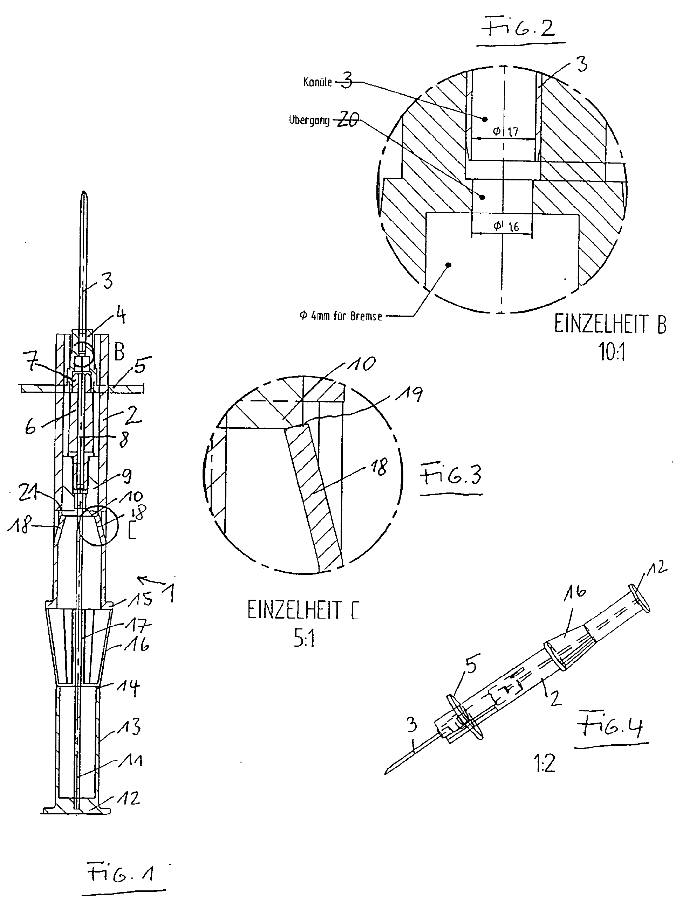

Die Implantatspritze 1 wird auf folgende Weise gehandhabt: zunächst wird die Spritzennadel 3 beispielsweise in die Bauchdecke eines Patienten eingestochen, bis die vordere Stirnkante des Nadelhalters 4 oder der Führungshülse 2 am Körper des Patienten anliegt. Dann wird das Distanzstück 16 entfernt. Daraufhin hintergreift der Benutzer das Griffstück 5 mit dem Mittelfinger und dem Zeigefinger und drückt mit dem Daumen auf die Endplatte 12, wodurch der Kolben 11 vorgeschoben wird, der das in dem Kanal 8 sitzende Präparat in den Kanal der Spritzennadel 3 bis zu dessen Spitze vorschiebt. Dabei wird das (nicht dargestellte) Präparat durch einen Übergang 20 (Figur 2) vorgeschoben, der einen etwas kleineren Durchmesser als das Präparat und der anschließende Kanal der Spritzennadel 3 hat, so daß das Präparat nicht von alleine aus der Spritzennadel 3 austreten kann.

Gegen Ende der Vorwärtsbewegung läuft die Hülse 13 auf die Federarme 18 auf und drückt diese radial nach außen, bevor die vordere Stirnkante 14 der Hülse 13 einen Anschlag 21 der Führungshülse 2 erreicht. Die elastisch nach außen gebogenen Federarme 18 geben nun die Rückwand der Spritzennadelanordnung frei.

Während der Benutzer weiterhin einen gegeneinander gerichteten Druck auf die Endplatte 12 und das Griffstück 5 ausübt, wird nun -nachdem die Hülse 13 auf den Anschlag 21 aufgetroffen ist- die Spritzennadelanordnung zurückgezogen, wobei die Spritzennadel das Präparat in dem Einstichkanal zurückläßt. Bei dieser Rückwärtsbewegung sollte die vordere Stirnkante der Führungshülse 2 in Berührung mit dem Körper des Patienten bleiben.

Da bei der vorstehend beschriebenen Betätigung der Implantatspritze 1 die Griffstellung nicht geändert werden muß und auf den Vorschub des Kolbens 11 unmittelbar das Zurückziehen der Spritzennadel 3 folgt, ist die Handhabung der Implantatspritze nicht nur besonders einfach, sondern der Vorgang benötigt zudem nur ein Minimum an Zeitaufwand.

## Patentansprüche

1. Implantatspritze mit einer Spritzennadelanordnung, die eine Spritzennadel (3), ein Griffstück (5) und ein Präparataufnahmeteil (6) enthält, die zur gemeinsamen axialen Bewegung in einer Führungseinrichtung (2) sitzen, und mit einem an einer Endplatte (12) befestigten Kolben (11), dessen Vorschub mit Hilfe einer an der Endplatte (12) befestigten und den rückwärtigen Endabschnitt des Kolbens (11) umgebenden Hülse (13) und eines Anschlags (21) an der Führungseinrichtung (2) derart begrenzt ist, daß zwischen der Austrittsöffnung der Spritzennadel (3) und dem Kopfende des Kolbens (11) ein Abstand verbleibt, der mindestens gleich der Länge eines abzugebenden Präparats ist, wobei die Spritzennadel (3) nach Aufhebung einer Blockiereinrichtung mittels des Griffstücks (5) mindestens um eine mit der Länge des Präparats übereinstimmende Strecke zurückziehbar ist,
**dadurch gekennzeichnet,**
**daß** die Blockiereinrichtung wenigstens einen Federarm (18) aufweist, der in seinem unbelasteten Ausgangszustand ins Innere der Führungseinrichtung (2) ragt und an der Spritzennadelanordnung anliegt, und daß die den Endabschnitt des Kolbens (11) umgebende Hülse (13) so angeordnet ist, daß sie beim Vorschub des Kolbens (11) den wenigstens einen Federarm (18) radial nach außen drückt, wodurch die Spritzennadelanordnung zum Zurückziehen freigegeben ist.

2. Implantatspritze nach Anspruch 1,
**dadurch gekennzeichnet, daß** der wenigstens eine Federarm im unbelasteten Ausgangszustand an einer rückwärtigen Stirnwand (10) der Spritzennadelanordnung anliegt.

3. Implantatspritze nach Anspruch 1 oder 2,
**dadurch gekennzeichnet, daß** der wenigstens eine Federarm (18) aus der Führungshülse (2) freigeschnitten ist.

4. Implantatspritze nach einem der Ansprüche 1 bis 3,
**dadurch gekennzeichnet, daß** das Griffstück (5) die Führungshülse (2) umgibt und mit Stegen mit einer innerhalb der Führungshülse (2) sitzenden Nadelhalterung (4) verbunden ist, wobei die Stege Längsschlitze der Führungshülse (2) durchgreifen.

## Claims

1. Implant syringe with a syringe needle arrangement consisting of a syringe needle (3), a gripping piece (5) and a preparation receiving part (6) which for common axial movement are seated in a guide means (2), and with a plunger (11) fixed on an end plate (12), the advancing movement of which is limited with the aid of a sleeve (13) which is fixed on the end plate (12) and surrounds the rear end portion of the plunger (11) and by a stop (21) in such a way that between the outlet opening of the syringe needle (3) and the head end of the plunger (11) a distance remains which is at least equal to the length of a preparation to be dispensed, wherein the syringe needle (3) can be retracted by means of the gripping piece (5) at least by a distance corresponding to the length of the preparation after release of a blocking means (18), **characterised in that** the blocking means has at least one spring arm (18) which in the unloaded initial state projects into the interior of the guide means (2) and rests on the syringe needle arrangement, and that the sleeve (13) surrounding the end portion of the plunger (11) is disposed so that during the advancing movement of the plunger the sleeve (13) it presses the at least one spring arm (18) radially outwards, whereby the syringe needle arrangement is released for retraction.

2. Implant syringe as claimed in Claim 1, **characterised in that** the at least one spring arm rests in the unloaded initial state on a rear end wall (10) of the syringe needle arrangement.

3. Implant syringe as claimed in Claim 1 or 2, **characterised in that** said at least one spring arm (18) is cut out from the guide sleeve (2).

4. Implant syringe as claimed in any one of Claims 1 to 3, **characterised in that** the gripping piece (5) surrounds the guide sleeve (2) and is connected by webs to a needle holder (4) seated inside the guide sleeve (2), the webs passing through longitudinal slots in the guide sleeve (2).

## Revendications

1. Seringue pour implant avec un ensemble d'aiguille de seringue, contenant une aiguille de seringue (3), une manette (5) et une partie de logement de la préparation (6) qui reposent dans une installation de guidage (2) en vue du mouvement axial commun, et avec un piston (11) fixé à une plaque d'extrémité (12) dont l'avance est limitée à l'aide d'un manchon (13) fixé à la plaque d'extrémité (12) et entourant le segment d'extrémité arrière du piston (11), et à l'aide d'une butée (21) contre l'installation de guidage (2), de telle sorte que, entre l'orifice d'échappement de l'aiguille de seringue (3) et l'extrémité du piston (11), un écart demeure qui a une longueur au moins identique à celle d'une préparation devant être délivrée, l'aiguille de seringue (3) étant rétractile après relèvement d'une installation de blocage au moyen de la manette (5) au moins d'une distance concordant avec la longueur de la préparation,
**caractérisée en ce que**
l'installation de blocage comprend au moins un bras à ressorts (18) qui ressort dans son état de sortie non chargé vers l'intérieur de l'installation de guidage (2), et est adjacent à l'ensemble d'aiguille de seringue, et **en ce que** le manchon (13) entourant le segment d'extrémité du piston (11) est disposé de telle sorte qu'il pousse radialement vers l'extérieur lors de l'avancée du piston (11) l'au moins un bras à ressorts (18), libérant l'ensemble d'aiguille de seringue en vue du retrait.

2. Seringue pour implant selon la revendication 1,
**caractérisée en ce que** l'au moins un bras à ressorts est adjacent, à l'état de sortie non chargé, à une paroi frontale arrière (10) de l'ensemble d'aiguille à ressorts.

3. Seringue pour implant selon la revendication 1 ou 2, **caractérisée en ce que** l'au moins un bras à ressorts (18) est libéré par découpage du manchon de guidage (2).

4. Seringue pour implant selon l'une des revendications 1 à 3,
**caractérisée en ce que** la manette (5) entoure le manchon de guidage (2) et est reliée avec des brides à un porte-aiguille (4) situé à l'intérieur du manchon de guidage (2), les brides traversant des fentes longitudinales du manchon de guidage (2).
